Europäisches Patentamt

European Patent Office

Office européen des brevets

(19)

(11) Veröffentlichungsnummer: **0 245 846 B1**

(12) **EUROPÄISCHE PATENTSCHRIFT**

(45) Veröffentlichungstag der Patentschrift:
02.01.91 Patentblatt 91/01

(51) Int. Cl.⁵ : **A61F 2/36**

(21) Anmeldenummer: 87106896.1

(22) Anmeldetag: 12.05.87

(54) **Prothesenteil sowie Verfahren zu seiner Herstellung.**

(30) Priorität: 16.05.86 DE 3616665
09.03.87 DE 3707518

(43) Veröffentlichungstag der Anmeldung:
19.11.87 Patentblatt 87/47

(45) Bekanntmachung des Hinweises auf die
Patenterteilung:
02.01.91 Patentblatt 91/01

(84) Benannte Vertragsstaaten:
CH DE FR GB IT LI

(56) Entgegenhaltungen:
EP-A- 0 169 976
DE-A- 3 536 894
DE-A- 3 536 895

(73) Patentinhaber: Copf, Franz, Dr.-med.
Eberhardstrasse 1
D-7000 Stuttgart 1 (DE)
Patentinhaber: Holz, Ulrich, Prof. Dr.med.
Lenbachstrasse 56
D-7000 Stuttgart 1 (DE)

(72) Erfinder: Copf, Franz, Dr.-med.
Eberhardstrasse 1
D-7000 Stuttgart 1 (DE)
Erfinder: Holz, Ulrich, Prof. Dr.med.
Lenbachstrasse 56
D-7000 Stuttgart 1 (DE)

(74) Vertreter: Ostertag, Reinhard et al
Patentanwälte Dr. Ulrich Ostertag Dr.
Reinhard Ostertag Eibenweg 10
D-7000 Stuttgart 70 (DE)

## Beschreibung

Die Erfindung betrifft ein orthopädisches Prothesenteil gemäß dem Oberbegriff des Anspruches 1, sowie ein Verfahren en seiner Herstellung.

Ein solches Prothesenteil ist in der DE-A-35 36 894 beschrieben. Bei diesem bekannten Prothesenteil sind die Verankerungspfeiler korkenzieherähnlich gebogen und stützen sich mit Abschnitten an der Wand einer Ausnehmung elastisch ab, welche im Skelettknochen erzeugt worden ist. Auf diese Weise kann sich das Verankerungsteil elastisch an die Knochenausnehmung anpassen, während dies bei älteren Schaftprothesen mit starrem Schaft nicht möglich war. In den Zwischenraum zwischen den einzelnen Verankerungspfeilern kann beim Heilungsprozeß Spongiosamaterial hineinwachsen, so daß das Prothesenteil ohne Verwendung von Knochenzement bleibend mit dem Skelettknochen verbunden wird.

Bei diesem bekannten Prothesenteil hat man jedoch bei sehr starken Belastungen nicht immer eine zufriedenstellende Kraftübertragung von dem Prothesenteil auf den Knochen, da das Spongiosamaterial, in welches die Verankerungspfeiler einheilen, verhältnismäßig weich ist und die maximale Belastbarkeit des Prothesenteiles von der Belastbarkeit der Grenzschicht zwischen der Oberfläche der Verankerungspfeiler und der Oberfläche des umgebenden Spongiosamateriales abhängt.

Es sind ferner Schaft-Prothesenteile bekannt, bei denen die Spongiosa vollständig aus einem Abschnitt eines Skelettknochens ausgeräumt wird und dort der Schaft des Prothesenteiles unter Verwendung von Polymethylmethacrylat (PMMA) festgeklebt wird. Dem PMMA wird zur Herstellung eines Knochenzementes in der Regel noch feingekörntes PMMA zugefügt. Siehe hierzu "Encyclopädie Naturwissenschaft und Technik", Verlag Moderne Industrie, 1979, Stichwort "Knochenzement".

Derartige einzementierte Schaft-Prothesenteile stellen im Hinblick auf die Kraftübertragung zunächst zufrieden, da vom starren Schaft auf die harte Außenschicht des Skelettknochens (Kortikalis) hohe Kräfte übertragen werden können. Allerdings ist die Klebverbindung doch wegen des sehr unterschiedlichen thermischen Ausdehnungskoeffizienten von Knochenzement und Prothesenwerkstoff und wegen der beim Aushärten erfolgenden Schrumpfung des Knochenzementes zuweilen nachteilig. Auch kann es aufgrund der chemischen Natur des Knochenzementes und durch das Aushärten desselben zu Gewebebeschädigungen kommen, und nicht bei allen Knochen ist im Einzelfalle eine gute Verankerung gewährleistet.

Durch die vorliegende Erfindung soll ein Gelenkprothesenteil gemäß dem Oberbegriff des Anspruches 1 so weitergebildet werden, daß eine noch bessere Krafteinleitung von den Verankerungspfeilern in die Spongiosa erhalten wird.

Diese Aufgabe ist erfindungsgemäß gelöst durch ein Prothesenteil gemäß Anspruch 1.

Bei dem erfindungsgemäßen Prothesenteil wird ein großes Volumen des betrachteten Skelettknochenabschnittes ausgeräumt. Der dieses Volumen ursprünglich abdeckende Abschnitt der Kortikalis wird durch eine Tragwand des Verankerungsteiles ersetzt, wobei sich eine Vielzahl stabförmiger Verankerungspfeiler von dieser Tragwand ins Knocheninnere erstreckt.

Beim Implantieren des erfindungsgemäßen Gelenkprothesenteiles wird ein der Außenkontur der VerankerungspfeilerAnordnung entsprechendes Spongiosavolumen aus dem Knochen ausgeräumt. Die entsprechende Menge an Spongiosamaterial wird in einer Spongiosamühle zermahlen und dann zwischen die Verankerungspfeiler eingefüllt. Im Verlaufe des Heilungsprozesses wachsen die verschiedenen Spongiosapartikel wieder zusammen und umwachsen die Verankerungspfeiler, welche erfindungsgemäß mit Verankerungsbunden ausgebildet sind

Nach dem Einwachsen des Verankerungsteiles hat man somit einen Kraftfluß vom Prothesenteil zum betrachteten Skelettknochen, welcher über eine sehr große Berührfläche über das Spongiosamaterial verläuft. Entsprechend nieder sind die lokalen Belastungen der Spongiosa. Die Spongiosa hat nach dem Heilungsprozeß wieder eine porige Struktur, was auch im Hinblick auf die Dämpfung von Stößen vorteilhaft ist. Bei mit Knochenzement fixierten Gelenkprothesenteilen ist dagegen die Verbindungsstruktur zwischen Prothesenteil und Knochen starr, so daß dort bei Stoßbelastungen hohe Beanspruchungen auftreten können. Diese können somit zur Deformierung des Knochens führen, woraus sich dann ein Lockern des Prothesenteiles ergibt. Hat sich dieses erst einmal gelockert, so führen die zunächst kleinen Relativbewegungen zu Reizbelastungen und zu sich weiter verschlechternden Fixationsbedingungen. Da bei Verwendung des erfindungsgemäßen Prothesenteiles die Verbindungsstruktur zwischen Prothese und Knochen die lebende Spongiosa umfasst, kann nachwachsende Spongiosa auch lokale Schwächen in der Verbindungsstruktur ausheilen ; der oben geschilderte circulus vitiosus ist ausgeräumt.

Da erfindungsgemäß die von der Tragwand des Prothesenteiles weglaufenden Verankerungspfeiler trabeculär orientiert sind (längs der Haupt-Kraftübertragungswege verlaufen), hat man auch nach dem Ausheilen im die Prothesenteil tragenden Knochen sehr ähnliche Kraftübertragungsverhältnisse wie im gesunden Knochen. Bei zementverankerten Prothesen sind dagegen die Kraftübertragungsverhältnisse völlig anders als im gesunden Knochen, und für derartige Verhältnisse ist der Knochen nicht ausgelegt. Es können sich somit zusätzliche Schwierigkeiten selbst noch an weiter vom Prothesenteil abliegenden Knochenabschnitten ergeben.

Die erfindungsgemäß auf den Verankerungspfeilern vorgesehenen Verankerungsbunde dienen einerseits zur Vergrösserung der Berührfläche zwischen dem Verankerungsteil des Prothesenteiles und der Spongiosa ; sie dienen zugleich als Mittel, welche eine Relativbewegung zwischen Verankerungspfeiler und Spongiosa verhindern und darüber hinaus als Mittel zum Einleiten von Kräften in das Spongiosamaterial in Richtung der trabeculären Traiectoren, in welcher Richtung die Spongiosa besonders belastungsfähig ist. Die oben genannten Vorteile werden mit einem erfindungsgemäßen Prothesenteil erhalten, wie es im Anspruch 1 angegeben ist.

Vorteilhafte Weiterbildungen der Erfindung sind in Unteransprüchen angegeben.

Die weiterbildungen der Erfindung gemäß den Ansprüchen 2 und 3 sind im Hinblick auf ein möglichst schonendes Einleiten der Kräfte in die Spongiosa von Vorteil

Mit der Weiterbildung der Erfindung gemäß Anspruch 4 wird erreicht, daß die nach Einheilen des Prothesenteiles an die Verankerungspfeiler herangewachsene Spongiosa keinen lokalen Belastungen ausgesetzt ist, die zu bleibender Beschädigung führen könnten, umgekehrt aber so starken Reizen ausgesetzt ist, daß sie sich ständig wie im gesunden Knochen regeneriert und hierdurch dynamisch fest gegen die Verankerungspfeiler gewachsen bleibt.

Derartige Belastungsverhältnisse sind in der Praxis in der Begel gewährleistet, wenn die Oberflächen-Bemessungsregel des Anspruches 5 erfüllt ist.

Mit der Weiterbildung der Erfindung gemäß Anspruch 6 wird erreicht, daß in der wiederhergestellten Spongiosa keine nennenswerten lokalen Querschnittsverminderungen der einzelnen säulenförmigen spongiosaerfüllten Zwischenräume zwischen den Verankerungspfeilern erhalten werden; es ergeben sich keine Teilvolumina der Spongiosa, welche starken kerbwirkungen ausgesetzt wären.

Auch die Weiterbildung der Erfindung gemäß Anspruch 7 ist im Hinblick auf eine gleichförmige mechanische Belastung der wiederhergestellten Spongiosa von Vorteil.

Ein Prothesenteil, wie es im Anspruch 8 angegeben ist, kann unter Verwendung eines externen Halteelementes für diejenige Zeit am Skelettknochen festgelegt werden, innerhalb welcher die Spongiosapartikel zusammen und gegen die Verankerungspfeiler wachsen.

Mit der Weiterbildung der Erfindung gemäß Anspruch 9 wird es möglich, ein Spongiosavolumen möglichst weit mit Verankerungspfeilern zu durchsetzen, was im Hinblick auf möglichst kleine lokale Belastungen der Spongiosa von Vorteil ist, wobei dann die Tragwand denjenigen Teil der Kompakta ersetzt, der zur Schaffung eines breiten Zuganges zum Spongiosavolumen entfernt werden muß.

Beim Einsetzen eines Prothesenteiles kann es sich als sachdienlich erweisen, an einzelnen Stellen des Knochens etwas weniger Spongiosa auszuräumen als bei der Planung der Operation zunächst vorgesehen. Bei einem Prothesenteil gemäß Anspruch 10 kann die lichte effektive Außenkontur in derartigen Fällen rasch angepasst werden, indem von den freitragenden Endabschnitten der Verankerungspfeile mit einer Zange ein entsprechend langes Stück abgeschnitten wird.

Mit der Weiterbildung der Erfindung gemäß Anspruch 11 wird erreicht, daß die Verankerungspfeiler ein sehr starken mechanischen Belastungen standhaltendes Fachwerk bilden. Auch im Hinblick auf die Herstellung des Prothesenteiles durch Gießen sind verbundene Verankerungspfeiler von Vorteil, da man so nur wenige Entlüftungskanäle benötigt.

Ein Prothesenteil, wie es im Anspruch 12 angegeben ist, ist gut an das am Knochenende zur Implantation zur Verfügung stehende Spongiosavolumen angepasst. Auf diese Weise erhält man eine besonders gute Kraftverteilung. Das olivenförmige Endstück des Prothesenteiles erleichtert ein Einführen der Prothese in das ausgeräumte Knochenende unter den in der Regel weniger guten Sichtbedingungen bei der Operation.

Auch die Weiterbildung der Erfindung gemäß Anspruch 13 ist im Hinblick auf ein leichteres Einführen des Prothesenteiles in den Knochen von Vorteil. Darüber hinaus erleichtert die gemäß Anspruch 13 vorgesehene Durchgangsbohrung des Endstückes das Ausformen des Prothesenteiles, wenn letzteres durch Gießen in einer Keramikform hergestellt wird.

Mit der Weiterbildung der Erfindung gemäß Anspruch 14 wird es möglich, die Kraftübertragung zwischen Prothesenteil und Spongiosa weiter zu vergleichmäßigen, da man eine noch größere Kontaktfläche zwischen den Verankerungspfeilern und der Spongiosa erhält.

Bei einem Prothesenteil gemäß Anspruch 15 kann man sekundäre Verankerungspfeiler mit kleinem Querschnitt auch unter Abstand von der Tragwand vorsehen, da das flüssige Prothesenmaterial beim Herstellen des Prothesenteils durch Gießen über die grösseren Querschnitt aufweisenden primären Verankerungspfeiler zugeführt werden kann. Mann erhält auf diese Weise sauber ausgebildete sekundäre Verankerungspfeiler.

Ein Prothesenteil gemäß Anspruch 16 kann als eine im wesentlichen gleichförmige offenporige Struktur angesehen werden, welche das Spongiosavolumen im Knochenende nach der Implantation im wesentlichen gleichförmig erfüllt, wobei die Haupt-Kraftübertragungslinien ebenso verlaufen wie beim natürlich gewachsenen Knochen. Dadurch, daß das Prothesenvolumen im wesentlichen gleichförmig mit Verankerungspfeilern durchsetzt ist, kann man auch das Innere des durch die Verankerungspfeiler insgesamt vorgegebenen Käfigs besonders einfach mit in einer Mühle auf etwa gleiche Korngröße gemahlener Spongiosa ausfüllen, wobei die gemahlene Spongiosa auch gut im Inneren des Prothesenteiles verbleibt, wenn die Korngröße auf den mittleren Abstand der Verankerungspfeiler angepasst ist. Man kann also die zerkleinerte Spongiosa schon vor der Implantation des Prothesenteiles in dieses einfüllen und zusammen mit dem Prothesenteil in das ausgeräumte Knochenende einsetzen. Dies erleichtert die Operation erheblich und macht es auch leichter, das Prothesenteil von einer Schmalseite des Knochenendes her einzusetzen, wobei sonst Schwierigkeiten bestünden, den gesamten zwischen den Verankerungspfeilern liegenden Raum wieder gleichförmig mit Spongiosamaterial auszufüllen. Das Einsetzen des Prothesenteiles von einer Schmalseite des Knochenendes her ist aber deshalb besonders vorteilhaft, weil am Knochen angreifende Muskelenden häufig auf der Breitseite des Knochenendes festgewachsen sind. Beim Einführen des Prothesenteiles von der Schmalseite her brauchen diese Muskelenden nicht abgetrennt zu werden.

Abmessungen der Verankerungspfeiler, wie sie im Anspruch 17 angegeben sind, haben sich im Hinblick auf die Kraftübertragung zwischen den Verankerungspfeilern und der Spongiosa einerseits und im Hinblick auf das Herstellen des Prothesenteiles durch Gießen von Biomaterial als vorteilhaft erwiesen.

Bei manchen Knochen ist ein Teil des beim Knochenende liegenden Spongiosavolumens im Einsatz auf Druck belastet, während ein anderer Teil des Spongiosavolumens auf Zug belastet ist. Derartigen Anwendungsfällen kann man mit einem Prothesenteil gemäß Anspruch 18 besonders gut Rechnung tragen, da die Verankerungspfeiler kegelstumpfförmige Verankerungsbunde tragen, welche sich je nach Ausrichtung besonders gut zur Aufnahme von Druck- oder Zugbelastungen eignen.

Stellt man erfindungsgemäße Prothesenteile gemäß Anspruch 19 her, so kann man auch solche Anordnungen von Verankerungspfeilern zu geringen Kosten realisieren, welche Hinterschneidungen aufweisen.

Dabei kann man gemäß Anspruch 20 in ähnlicher Weise vorgehen, wie dies an sich vom Gießen von Glocken her bekannt ist.

Das im Anspruch 21 angegebene Verfahren erlaubt eine exakte Berücksichtigung der Form desjenigen Knochens, welcher mit dem Prothesentiel versehen werden soll. Der Operateur kann so mit einem für den speziellen Fall optimal angepassten Prothesenteil arbeiten.

Die Weiterbildungen der Erfindung gemäß den Ansprüchen 22 und 23 sind im Hinblick auf ein einfaches maschinelles Ableiten des Knochenmodelles von Standard-Röntgenaufnahmen des mit dem Prothesenteil zu versehenden Knochens von Vorteil.

Mit der Weiterbildung der Erfindung gemäß Anspruch 24 ist es möglich, diejenigen Informationen über die Geometrie des betrachteten Knochens, welche man nur durch eine große Anzahl zusätzlicher Röntgenaufnahmen ermitteln könnte, die darüber hinaus kompliziert auszuwerten wären, aus einem in einem Massenspeicher abgelegten Vorrat von Knochenformdaten abzurufen. Bezüglich der Haupt-Randkonturen entspricht somit das hergestellte Knochenmodell exakt den in der Regel zwei in zueinander senkrechten Richtungen aufgenommenen Röntgenbildern, während bezüglich der weniger wichtigen Einzelheiten der Querschnittsänderung des Knochens der Rechner eine Ergänzung aus ihm zur Verfügung stehenden Daten vornimmt.

Gemäß Anspruch 25 erzeugt der Rechner auch automatisch ein Modell für die Tragwand des Prothesenteiles derart, daß diese Tragwand einen den Spongiosaraum verschliessenden, eine bündige Fortsetzung der Kompakta des Knochens darstellenden Deckel bildet.

Gemäß Anspruch 26 wird vom Rechner zusätzlich ein Verankerungspfeiler-Belegungsplan erstellt, wobei das entsprechende Rechnerprogramm grob gesprochen so arbeitet, daß es die Verankerungspfeiler längs trabeculärer Traiectoren ausrichtet und in senkrecht auf diesen Traiectoren liegenden Ebenen im wesentlichen gleich verteilt.

Die Weiterbildung der Erfindung gemäß Anspruch 27 erleichtert das Umsetzen des so erzeugten Belegungsplanes in das Modell, da die Fußpunkte der Verankerungspfeiler mechanisch vorgegeben sind.

Nachstehend wird die Erfindung anhand von Ausführungsbeispielen unter Bezugnahme auf die Zeichnung näher erläutert.

In dieser zeigen :

Figur 1 : eine Frontansicht des oberen Endes eines Oberschenkelknochens mit einem eingesetzten Prothesenteil in einer ersten Phase des Einsetzvorganges ;

Figur 2 : eine ähnliche Ansicht wie Figur 1 in einer zweiten Phase der Prothesenimplantation ;

Figur 3 : eine ähnliche Ansicht des Oberschenkelknochens nach Beendigung der Implantation und vorläufiger Fixierung des Prothesenteiles ;

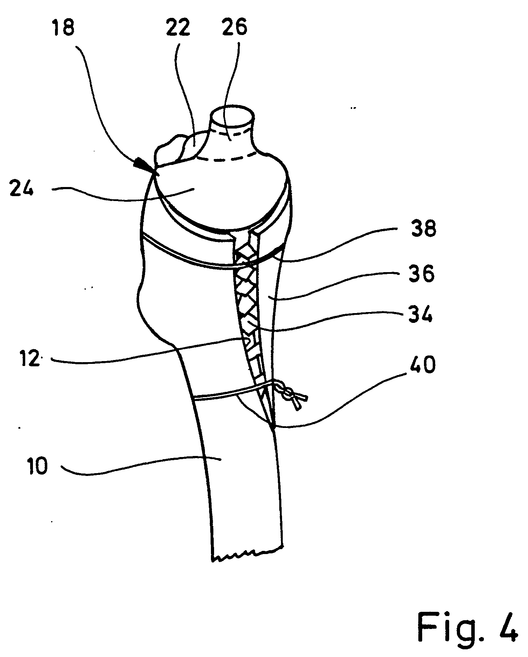

Figur 4 : eine Ansicht des Oberschenkelknochens nach Implantation des Prothesenteiles in Figur 3 von links ;

Figur 5 : eine schematische Ansicht einer Vorrichtung, die ein maßstabsgetreues Modell des mit dem Prothesenteil zu versehenden Knochenabschnittes herstellt ;

Figur 6 : eine Aufsicht auf einen Ausschnitt aus einem Endlosmaterial, wie es bei der Herstellung eines Prothesenmodelles verwendet wird ;

Figur 7 : eine Aufsicht auf ein teilweise fertiggestelltes Prothesenmodell ;

Figur 8 : einen Schnitt durch den Verankerungsabschnitt eines Prothesenteiles in einer senkrecht zu den Achsen der Verankerungspfeiler verlaufenden Ebene ;

Figur 9 : eine Schnitt durch das Prothesenteil nach Fig. 1 längs dessen Mittelebene ;

Figur 10 : eine seitliche Ansicht eines abgewandelten Oberschenkelknochen-Prothesenteiles ; und

Figuren 11 bis 16 : Ausschnitte aus Verankerungspfeilern mit abgwandelter Geometrie in größerem Maßstab.

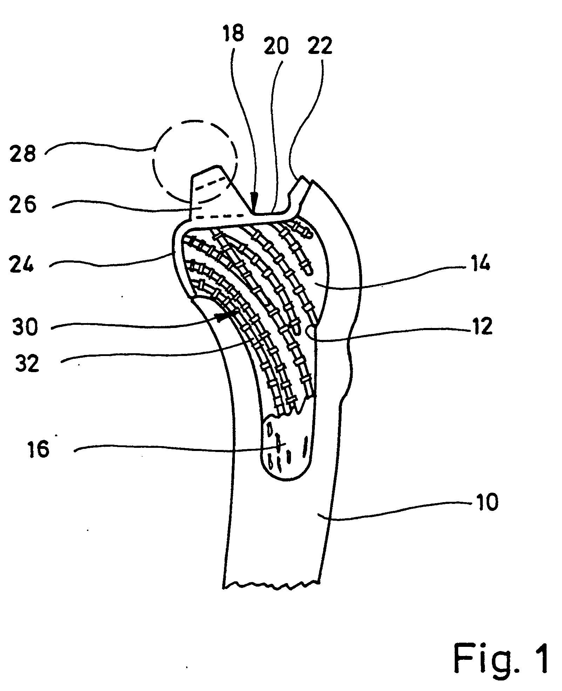

In Figur 1 ist ein oberer Endabschnitt eines Oberschenkelknochens 10 wiedergegeben, der für die Implantation eines Hüftgelenk-Prothesenteiles vorbereitet ist. Hierzu ist die Gelenkkugel und der Gelenkhals vollständig entfernt worden, und auf der Knochenvorderseite ist die kompakte feste Außenschicht (Kompakta) ausgesägt, so daß ein Fenster 12 erhalten wird. In dem hinter dem Fenster 12 liegenden Abschnitt ist die Spongiosa aus dem Oberschenkelknochen 10 weitgehend bis zur Kompakta hin ausgeräumt, so daß ein Hohlraum 14 geschaffen wird. In Figur 1 sind bei 16 noch Reste der Spongiosa sichtbar. Man erkennt dort die schwammähnlichen Maschenräume.

In das obere Ende des Oberschenkelknochens 10 ist ein insgesamt mit 18 bezeichnetes Prothesenteil eingesetzt. Zu letzterem gehört eine Tragwand 20, welche im wesentlichen die Form eines Doppelwinkels hat, wobei der eine Winkelschenkel 22 zum Trochanter major verläuft, während der zweite Winkelschenkel 24 einen bündigen Anschluß zum Adamschen Bogen herstellt. Die Tragwand 20 stellt somit eine Kappe dar, welche das obere Ende des Hohlraumes 14 bündig verschließt.

Auf der Oberseite ist an die Tragwand 20 ein Zapfen 26 angeformt, an welchem eine Gelenkkugel 28 mechanisch befestigbar ist, welche in Figur 1 gestrichelt angedeutet ist. Diese Gelenkkugel arbeitet mit einer am Becken zu implantierenden Gelenkpfanne zusammen, welche in der Zeichnung nicht wiedergegeben ist. Die Achse des Zapfens 26 ist so angestellt, daß man für die Gelenkkugel 28 insgesamt eine Valgus-Stellung, also eine Lage des Gelenkpunktes, welche der Achse des Knochenschaftes benachbart ist, sowie den gewünschten Antetorsionswinkel erhält.

Von der Innenseite der Tragwand 20 verläuft eine Vielzahl angeformter Verankerungspfeiler 30 nach innen in den Hohlraum 14 hinein. Wie aus der Zeichnung ersichtlich, ist die Grunderstreckungsrichtung der Verankerungspfeiler 30 im wesentlichen parallel zur Achse des Zapfens 26 und liegt somit in der Hauptbelastungsrichtung (Richtung der trabeculären Traiectoren). Entsprechend dem Verlauf dieser Traiectoren sind die einzelnen Verankerungspfeiler 30 etwas gekrümmt, wobei ein Teil dieser Verankerungspfeiler in einem dem Pfeilerfuß benachbarten Abschnitt etwas stärker gekrümmt ist, so daß diese Verankerungspfeiler an dem zweiten Winkelschenkel 24 festgelegt werden können. Die freien Enden der Verankerungspfeiler 30 enden unter Abstand vor der in Figur 1 rechts gelegenen aus Kompakta bestehenden Wand des Hohlraumes 14.

Wie die Zeichnung zeigt, haben die Verankerungspfeiler 30 jeweils eine Vielzahl axial aufeinanderfolgender Verankerungsbunde 32, die einen abgerundeten Rand aufweisen.

Die Verankerungspfeiler 30 sind unter im wesentlichen gleichem Abstand voneinander über den Hohlraum 14 verteilt, so daß zwischen ihnen im wesentlichen gleichen Querschnitt aufweisende gestreckte oder säulenförmige Freiräume verbleiben.

Die ringförmigen Stirnflächen der Verankerungsbunde 32, die Dicke der Verankerungsbunde 32, der axiale Abstand der Verankerungsbunde 32, die Dicke der Kernabschnitte der Verankerungspfeiler 30, sowie die Länge, die Anzahl und der Abstand der Verankerungspfeiler 30 sind insgesamt so bemessen, daß die lokalen Belastungen nach Einheilung des Prothesenteiles in der an die Verankerungspfeiler herangewachsenen Spongiosa nicht so groß ist, daß eine bleibende Schädigung der Spongiosa eintritt, umgekehrt aber auch nicht so klein ist, daß die für die ständige Erneuerung der Spongiosa erwünschten mechanischen Reize und kleineren Zerstörungen an einzelnen Maschen ausbleiben.

Hierzu sollten in der Spongiosa gelegentlich kurzfristige Belastungen auftreten, die etwa 120% bis 140% der Druckfließgrenze des jeweils betrachteten Spongiosatyps betragen. Für längerdauernde Belastungen kann die Belastung etwa 60% bis 80% der Druckfließgrenze betragen. Für die im Oberschenkelknochen enthaltene Spongiosa mit einer Druckfließgrenze von 2,0 N/mm² bedeutet dies, daß an den Verankerungsbunden 32 Flächendrucke von maximal 2,8 N/mm² auftreten sollen. Entsprechend ist die Ringfläche und Anzahl der Verankerungsbunde 32 zu wählen.

Als Anhaltspunkt kann in diesem Zusammenhange gelten, daß die Gesamtoberfläche der Verankerungspfeiler (Mantelflächen der Kernabschnitte, Stirnflächen und Umfangsflächen der Verankerungsbunde) etwa 130% bis etwa 270% der Wandfläche des Hohlraumes 14 betragen soll, aus welchem die Spongiosa zur Implantierung des Prothesenteiles entfernt wird.

Das Prothesenteil 18 ist ein einstückiges Gußteil, welches aus einem gewebeverträglichen Metall, z.B. CoCrMo-Biomaterial hergestellt ist.

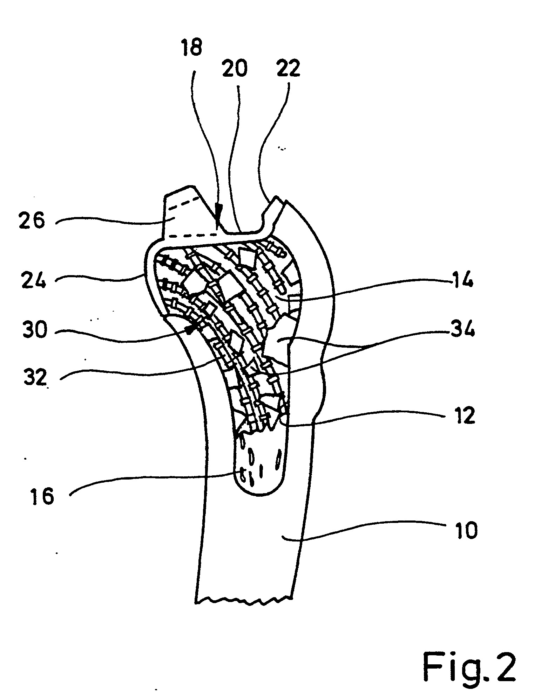

Figur 2 zeigt eine zweite Phase der Prothesenimplantation, in welcher die Zwischenräume zwischen den Verankerungspfeilern 30 mit gemahlenem Spongiosamaterial, welches zuvor dem Knochen zur Bildung des Hohlraumes 14 entnommen wurde, ausgefüllt wird. Der besseren Darstellung halber sind in Figur 2 z.B. bei 34 etwas grössere Spongiosapartikel dargestellt, als sie in der Praxis zur möglichst guten Ausfüllung der Zwischenräume zwischen den Verankerungspfeilern 30 verwendet würden.

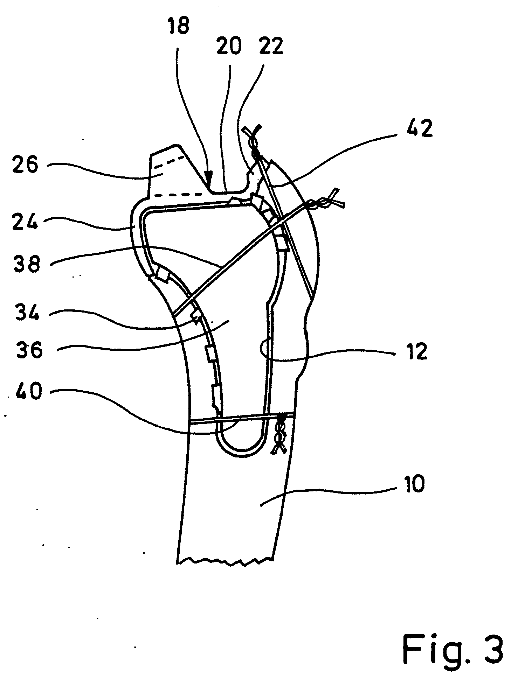

Figur 3 zeigt den Oberschenkelknochen nach Ausfüllen der Zwischenräume zwischen den Verankerungspfeilern 30 mit Spongiosapartikeln und nach Verschliessen des Fensters 12 durch das zuvor ausgesägte Kompaktastück 36. Das Kompaktastück 36 wird durch zwei Drähte 38, 40 aus Biomaterial in seiner Lage fixiert. Ein weiterer Draht 42, der in der Rinne zwischen dem ersten Winkelschenkel 22 und dem Zapfen 26 positioniert ist, dient zum vorläufigen Fixieren des Prothesenteiles 18 am Oberschenkelknochen so lange, bis das zwischen die Verankerungspfeiler 30 eingefüllte Spongiosamaterial zusammengewachsen und gegen die Außenflächen der Verankerungspfeiler 30 gewachsen ist. Dieses Festwachsen des Prothesenteiles 18 ist nach etwa 8 Wochen so weit fortgeschritten, daß das Prothesenteil 18 fest sitzt. Nach einem Jahr sind die Verankerungspfeiler 30 solide umwachsen.

Bei solide eingewachsenem Prothesenteil sind die Verankerungsbunde 32 in der Spongiosa eingewachsen, so daß keine Relativbewegung zwischen den Verankerungspfeilern 30 und der Spongiosa in Pfeilerlängsrichtung mehr möglich ist. Da man eine große Berührfläche zwischen der Spongiosa und den Verankerungspfeilern hat, können auch große statische Belastungen sicher vom Prothesenteil 18 auf den Knochen übertragen werden, wobei diese Übertragung über die poröse Struktur aufweisende Spongiosa erfolgt, so daß eine Dämpfung von Stoßbelastungen erhalten wird.

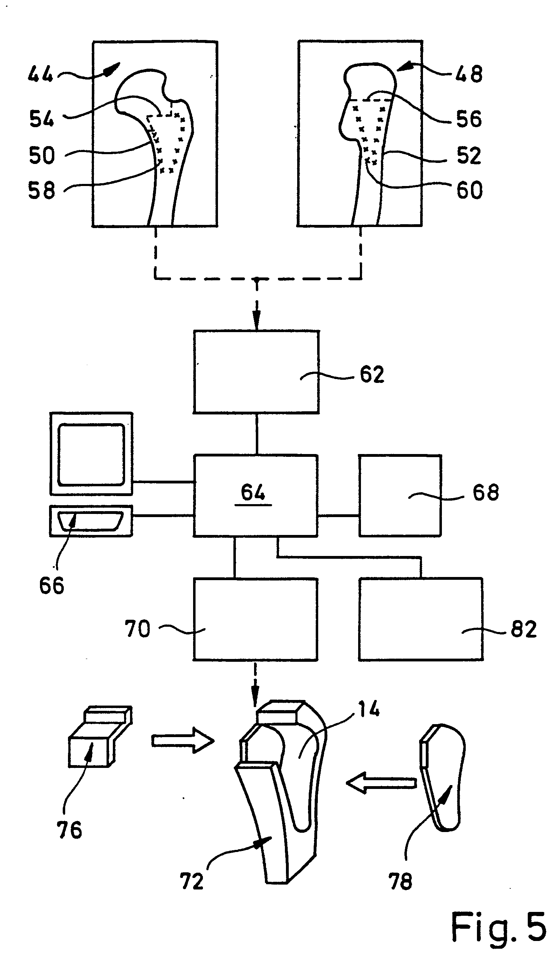

Nachstehend wird unter Bezugnahme auf die Figuren 5-8 die Herstellung des oben beschriebenen Prothesenteiles 18 erläutert.

Um die Geometrie des Prothesenteiles 18 für jeden Einzelfall optimal anpassen zu können, wird das Prothesenteil 18 für den speziellen Anwendungsfall eigens gefertigt. Hierzu wird von zwei Röntgenaufnahmen 44, 48 ausgegangen, welche das mit dem Prothesenteil 18 zu versehende obere Ende des Oberschenkelknochens von vorne gesehen bzw. von der Beckeninnenseite her gesehen zeigen. Diese beiden zueinander senkrechten Aufnahmen des oberen Endes des Oberschenkelknochens geben schon eine recht gute Information über die räumlichen geometrischen Verhältnisse.

In den beiden Röntgenaufnahmen 44, 48 ist die Randkontur des Knochens durch eine Linie 50 bzw. 52 vorgegeben. Zusätzlich hat der Operateur diejenige Linie, an welcher er den Gelenkkopf abtrennen will, durch gestrichelte Linien 54, 56 bezeichnet. Durch Kreuze markierte Linien 58, 60 markieren dasjenige Volumen im oberen Knochenendabschnitt, in welchem der Operateur die spongiosa auszuräumen beabsichtigt, um den Hohlraum 14 zu schaffen. Dieser Raum steht insgesamt für die Verankerungspfeiler zur Verfügung.

Die Linien 50-60 werden über ein Digitalisierungsgerät 62 in einen programmierbaren Rechner 64 eingegeben, welcher mit einem Sichtgerät 66 und einem Festplattenspeicher 68 zusammenarbeitet.

Ausgangsseitig ist der Rechner 64 mit einer numerisch gesteuerten Werkzeugmaschine 70 verbunden, welche aus einem Materialblock ein 1:1-Modell des oberen Endes des Oberschenkelknochens herausfräst, in welchem der Hohlraum 14 maßstäblich vorgesehen ist. Dieses Modell ist bei 72 perspektivisch wiedergegeben. Zur besseren Verständlichkeit ist hier angenommen, daß das Knochenmodell 72 ausschließlich aus den den Linien 50-60 entnehmbaren Infomationen abgeleitet ist; senkrecht zur Achse des Knochenschaftes gelegte Querschnitte durch das Knochenmodell 72 sind somit im wesentlichen rechteckig. Wo für die Praxis ein solches Modell zu grob ist, kann es in der weiter unten genauer beschriebenen Art und Weise verfeinert werden.

Zusätzlich zum Knochenmodell 72 erzeugt die Werkzeugmaschine 70 unter Steuerung durch den Rechner 64 noch ein Modell 76 der Tragwand 20 des Prothesenteiles 18, welches einen das obere Ende des Knochenmodelles 72 bündig verschliessenden Deckel darstellt, sowie einen seitlichen Deckel 78, welcher den Hohlraum 14 verschließt und ein Modell des Kompaktastückes 36 darstellt.

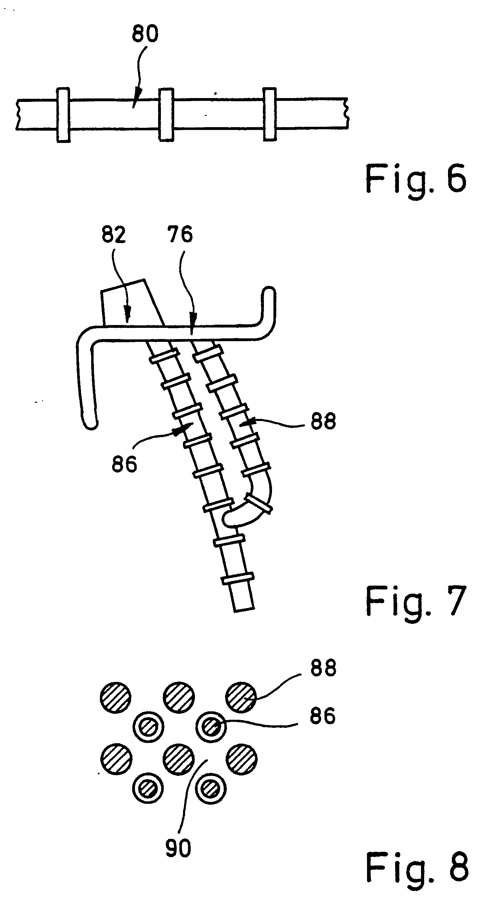

Ausgehend von dem Tragwandmodell 76 und dem Knochenmodell 72 kann dann ein 1:1-Modell des Prothesenteiles 18 hergestellt werden, indem das Tragwandmodell 76 mit 1:1-Modellen der Verankerungspfeiler 30 bestückt wird, was z.B. ausgehend von dem in Figur 6 in vergrössertem Maßstabe wiedergegebenen Endlosmaterial 80 erfolgen kann. Dieses Pfeilermodellmaterial kann in einem kontinuierlichen Verfahren hergestellt sein, man kann auch auf ein entsprechendes Kernmaterial einzelne Modellringe für die Verankerungsbunde 32 im Preßsitz aufschieben oder dort festkleben.

Der Rechner 64 ist ausgangsseitig ferner mit einem Plotter 82 verbunden, welcher unter Berücksichtigung des Querschnittes der einzelnen Verankerungspfeiler 30, unter Berücksichtigung der Neigung des Zapfens 26 und unter Berücksichtigung des gewünschten Abstandes zwischen den Verankerungspfeilern 30 einen Belegungsplan für die Verankerungspfeilermodelle erzeugt. Dies kann z.B. in Form zweier seitlicher Ansichten des Modelles erfolgen, welche den Betrachtungsrichtungen der beiden Röntgenaufnahmen 44 und 48 entsprechen. Zusätzlich kann der Rechner 64 die Werkzeugmaschine 70 derart steuern, daß in das Tragwandmodell 76 an den Fußpunkten der Pfeilermodelle Sackbohrungen eingebohrt werden, deren Neigung der Steigung der Verankerungspfeiler 30 am Fußpunkt entspricht.

Figur 7 zeigt ein Tragwandmodell 76, auf welchem ein Zapfenmodell 84 angebracht ist und welches schon zwei Pfeilermodelle 86, 88 trägt. Weitere Pfeilermodelle müssen noch nach dem vom Rechner über den Plotter 82 ausgegebenen Belegungsplan angebracht werden.

Wie aus Figur 7 ersichtlich, sind die Pfeilermodelle 86, 88 so von dem endlosen Pfeilermodellmaterial 80 abgelängt, daß die den Verankerungsbunden 32 entsprechenden Modellbunde um eine halbe Teilung gegeneinander versetzt sind. Auf diese Weise werden weitgehend in Umfangsrichtung geschlossene Kerbenanordnungen um die Spongiosasäulen herum verhindert, welche später zwischen den Verankerungspfeilern 30 wachsen.

Bei dem in Figur 7 gezeigten Modell ist in Abwandlung des obenstehend beschriebenen Prothesenteiles 18 das Pfeilermodell 88 an seinem unteren Ende zum Pfeilermodell 86 hingebogen und mit letzterem verbunden, so daß man zusätzlich auch eine Querversteifung im Pfeilerverbund erhält.

Figur 8 zeigt einen transversalen Schnitt durch die Pfeilermodellanordnung, aus welchem man erkennt, daß die verschiedenen Pfeilermodelle gleichverteilt sind, so daß zwischen ihnen im wesentlichen gleiche Querschnittsfläche aufweisende Zwischenräume 90 verbleiben, welche von Spongiosamaterial ausgefüllt werden können. Die Schnittebene von Figur 8 ist so gewählt, daß sie durch den einen Satz von Bunden (den der Pfeilermodelle 88) hindurch laufen, während die Bunde des anderen Satzes von Pfeilermodellen außerhalb der Zeichenebene liegen.

Das durch das Tragwandmodell 76, das Zapfenmodell 84 und die verschiedenen Pfeilermodelle 86, 88 gebildete Modell des Prothesenteiles 18 ist insgesamt aus einem Material gefertigt, welches bei mäßiger Wärmeeinwirkung flüssig wird oder sich verflüchtigt oder zersetzt. Man kann dann ausgehend vom Prothesenmodell dadurch eine Negativ-Gießform herstellen, daß man eine Sandform herstellt und nach deren Aushärten das Modellmaterial durch eine Wärmebehandlung entfernt. In die Gießform wird dann das flüssige Biomaterial hineingegossen, und nach seinem Erstarren wird das Gußstück aus der Gießform herausgenommen und gereinigt, wobei der zwischen den Verankerungspfeilern verbleibende Formsand gründlich entfernt wird, z.B. durch Sandstrahlen. Anschliessend wird der Rohling soweit erforderlich noch mechanisch bearbeitet. z.B. entgratet. Hieran kann sich dann eine zusätzliche Oberflächenbehandlung anschließen, z.B. ein Polieren und eine Oberflächenbeschichtung mit Titan.

Der oben beschriebene Herstellungsvorgang kann bezüglich der Herstellung des Knochenmodelles wie folgt verfeinert werden:

Auf dem Festplattenspeicher 68 werden die dreidimensionalen geometrischen Daten verschiedener charakteristischer Typen von Oberschenkelknochen abgelegt, zum Beispiel in Form der Randkonturen in Schaftlängsrichtung aufeinanderfolgenden Schnitte, also transversalen Schnitten durch den Knochen. Der Rechner prüft dann für die verschiedenen abgelegten Knochentypen, welcher Typ durch eine Ähnlichkeitstransformation (lineare Dehnung oder Kompression) am besten in Einklang mit den digitalisierten Randkonturen der Röntgenaufnahmen 44, 48 gebracht werden kann. Dies kann zum Beispiel unter Betrachtung der mittleren quadratischen Abweichung zwischen den durch Digitalisierung erhaltenen Randkonturlinien 50, 52 und den entsprechenden Linien der im Festplatzspeicher 68 abgelegten Knochentypen erfolgen. Von dem so als am besten übereinstimmend ermittelten Knochentyp werden dann zur Herstellung des Knochenmodelles 72 diejenigen Daten übernommen, welche die Abrundungen bei den Kanten des Knochenmodelles 72 ermöglichen. Man erhält so ein verbessertes Knochenmodell 72, welches auch in Einzelheiten mit dem mit dem Prothesenteil 18 zu versehenden Oberschenkelknochen übereinstimmt.

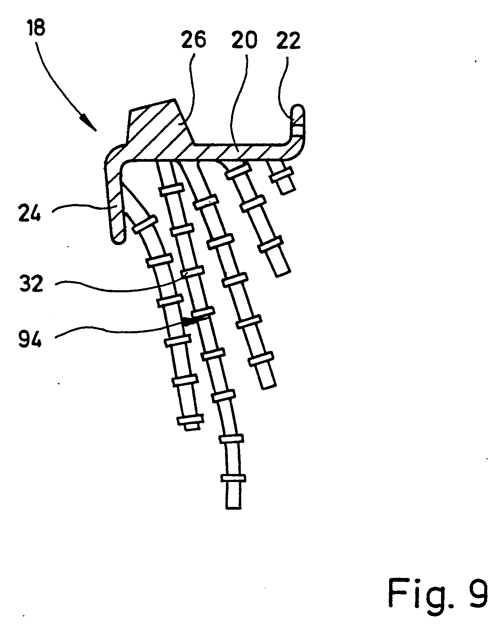

Figur 9 zeigt einen Schnitt durch die Mittelebene des Prothesenteiles 18, wobei jedoch der besseren Übersichtlichkeit halber nur die ersten hinter der Zeichenebene liegenden der Verankerungspfeiler gezeigt sind. Man erkennt im Winkelschenkel 22 eine Öffnung 92, durch welche der zur Zuggurtung dienende Draht 42 gezogen werden kann, der seinerseits in einer Durchgangsbohrung in Oberschenkelknochen 10 festgelegt ist.

Bei einem praktischen Ausführungsbeispiel eines Hüftgelenksprothesenteiles wie oben beschrieben können die Verankerungspfeiler 32 z.B. folgende Abmessungen aufweisen, wenn das Prothesenteil aus CoCrMo-Biomaterial gegossen ist:

- Kerndurchmesser der Verankerungspfeiler 2 mm
- Durchmesser der Verankerungsbunde 4 mm
- axialer Abstand der Verankerungsbunde 6 mm
- Abstand der Verankerungspfeiler in dem Bereich, in dem sie etwa parallel zueinander verlaufen 8 mm
- Länge der Verankerungsbunde gemäß im Hohlraum 14 verfügbaren Raum (längste Verankerungspfeiler ragen noch in den obersten Teil des Knochenschaftes)
- Neigung der Verankerungsbunde in Entfernung vom Fußpunkt in etwa gemäß Neigung der Achse des Zapfens 26 (längste Verankerungspfeiler im untersten Abschnitt zur Achse des Knochenschaftes hin abgekrümmt (Fortsetzung der in Fig. 1 dargestellten längsten Verankerungspfeiler)
- Dicke der Verankerungsbunde 1 mm

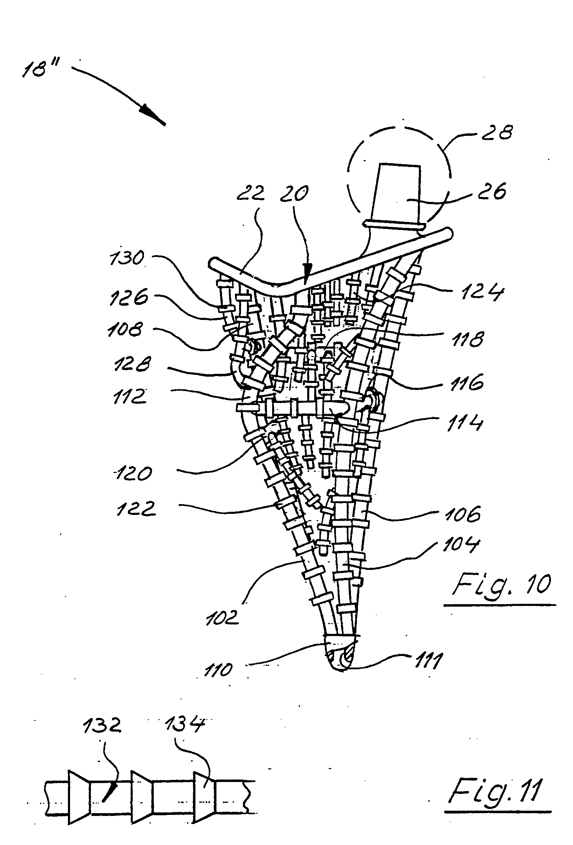

Bei der in Figur 10 gezeigten abgewandelten Gelenkprothese, die für einen linken Oberschenkelknochen bestimmt ist, sind Prothesenteile, die obenstehend schon beschrieben wurden, wieder mit denselben Bezugszeichen versehen.

Die abgewinkelte Tragwand 20 trägt vier großen Kerndurchmesser aufweisende primäre Verankerungspfeiler 102, 104, 106 und 108, deren freie Enden mit einem Prothesen-Endstück 110 zusammengegossen ist, welches im wesentlichen die Form einer Olive hat und außen glatt ist. Die primären Verankerungspfeiler 102-108 bilden so eine Eifel-Turm-ähnliche Rahmenkonstruktion, wobei die einzelnen Verankerungspfeiler zusammen einen stark belastbaren Käfig bilden, dessen transversaler Querschnitt sich von der Tragwand 20 zum Endstück 110 hin verkleinert. Die einzelnen Verankerungspfeiler sind so geschwungen, daß sie das im Ende des Oberschenkelknochens ausräumbare Spongiosavolumen bis nahe zum Rand hin erfüllen können. So hat z.B. der Verankerungspfeiler 102 einen gekrümmten Pfeilerabschnitt 112, um sich dem Spongiosa-erfüllten Raum besonders gut anpassen zu können.

Die im wesentlichen in Prothesenlängsrichtung verlaufenden primären Verankerungspfeiler 102-108 sind durch im wesentlichen im Umfangsrichtung verlaufende Verankerungspfeiler 114, 116, 118 und 120 verbunden, welche in unterschiedlicher Höhe liegen.

Die Verankerungspfeiler 102-108 und 114-120 haben bei einem praktischen Ausführungsbeispiel jeweils einen Kerndurchmesser von 3,5 mm, während auf ihnen angeordnete Verankerungsbunde 122 einen Außendurchmesser von 4,8 mm haben und unter einem Abstand von 3 mm aufeinander folgen. Die axiale Abmessung der Verankerungsbunde beträgt jeweils etwa 0,6 mm.

Auf der Tragwand 20 ist ferner eine Vielzahl demgegenüber dünner sekundärer Verankerungspfeiler 124 vorgesehen, die auch verglichen mit den primären Verankerungspfeilern kurze Länge haben. Bei einem praktischen Ausführungsbeispiel beträgt die Länge der sekundären Verankerungspfeiler 124 etwa 15-25 mm, wobei auch die sekundären Verankerungspfeiler 124 längs trabeculärer Trajektorien ausgerichtet sind und einige dieser sekundären Verankerungspfeiler 124 gekrümmte Abschnitte aufweisen können, wie bei 126 gezeigt, damit sie der Kontur des Spongiosavolumens des Knochenendes besser angepasst sind, und wobei auch die Enden einiger der sekundären Verankerungspfeiler 124 mit denen anderer sekundärer Verankerungspfeiler oder mit einem primären Verankerungspfeiler vergossen sein können, wie bei 128 gezeigt.

Um auch den unterhalb der freien Enden der von der Tragwand 20 getragenen sekundären Verankerungspfeiler 124 liegenden Teil des durch die primären Verankerungspfeiler 102-108 begrenzten Fachwerkes mit sekundären Verankerungspfeilern erfüllen zu können, kann man derartige sekundäre Verankerungspfeiler auch von den primären Verankerungspfeilern 102-108 oder von den in Umfangsrichtung verlaufenden starken verankerungspfeilern 114-120 ausgehend vorsehen. Im Hinblick auf das gleichförmige Erfüllen des durch die Verankerungspfeiler 102-108 vorgegebenen Käfigs mit sekundären Verankerungspfeilern 124 ist es vorteilhaft, die in Umfangsrichtung verlaufenden Verankerungspfeiler 114-120 in Prothesenlängsrichtung zu versetzen, wie in der Zeichnung dargestellt.

Das in Figur 10 gezeigte Prothesenteil besteht somit aus einem die Außenkontur vorgebenden, dem Spongiosavolumen des Knochenendes in der Form angepassten Käfig, dessen Inneres mit sekundären Verankerungspfeilern 124 im wesentlichen gleichförmig ausgefüllt ist. Auf diese Weise erhält man eine sehr große Berührfläche zwischen dem Prothesenteil und der nach der Implantation fest zwischen die primären und sekundären Verankerungspfeiler hineingewachsenen regenerierten Spongiosa. Entsprechend erhält man eine sehr gleichförmige und schonende Krafteinleitung in das Knochenende. Da wie bei den oben beschriebenen Ausführungsbeispielen keinerlei Zement oder Klebstoff benötigt wird, die Verankerung des Prothesenteiles rein biomechanisch erfolgt und sich somit laufend erneuert und ergänzt, ist auch langfristig ein guter Sitz des Prothesenteiles gewährleistet.

Bei einem praktischen Ausführungsbeispiel des in Figur 10 gezeigten Prothesenteiles haben die sekundären Verankerungspfeiler einen Kerndurchmesser von 2,5 mm und einen Außendurchmesser ihrer Verankerungsbunde 130 von 4 mm, wobei die Verankerungsbunde in Pfeilerlängsrichtung wieder einen Abstand von etwa 3 mm aufweisen. Noch schwächer dimensionierte sekundäre Verankerungspfeiler, die einen Teil der sekundären Verankerungspfeiler 124 ersetzen können, haben einen Kerndurchmesser von 2,2 mm, einen Außendurchmesser der Verankerungsbunde von 3 mm und einen Abstand der Verankerungsbunde von 3 mm. Der Abstand dieser Verankerungspfeiler von einander beträgt 6 bis 8 mm.

Dadurch, daß man eine sehr große Anzahl sekundärer Verankerungspfeiler hat, hat man zugleich auch viele Haltemöglichkeiten für dem Knochenende entnommenes gemahlenes Spongiosamaterial, welches zusammen mit dem Prothesenteil in das Knochenende eingesetzt wird und dort wieder zu einem zusammenhängenden Spongiosavolumen zusammenwachsen soll. Das in Figur 10 gezeigte Prothesenteil kann als linearer Schwamm angesehen werden, welcher gemahlene Spongiosa gut zwischen den sekundären Verankerungspfeilern 124 halten kann. Dies ermöglicht es, die gemahlene Spongiosa schon vor dem Implantieren des Prothesenteiles zwischen die sekundären Verankerungspfeiler 124 einzufüllen und das so gefüllte Prothesenteil in das Knochenende einzuführen.

Diese Art des Vorgehens macht es nicht notwendig, größere Mengen gemahlener Spongiosa nach Einsetzen des Prothesenteils zwischen die Verankerungspfeiler 124 zu stopfen, und man braucht somit auch keine große Zugangsöffnung zur Implantationsstelle. Das in Figur 10 gezeigte Prothesenteil eignet sich somit besonders gut zum Einsetzen von der Schmalseite des Knochenendes her, und dies ermöglicht es, die Implantation ohne das Abtrennen an der Knochenbreitseite angewachsener Muskelenden durchzuführen. Dies kann die Heilung erheblich beschleunigen.

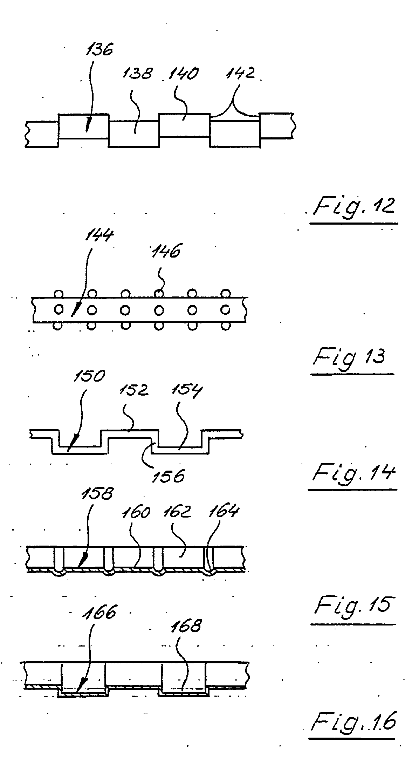

Figur 11 zeigt einen Ausschnitt aus einem abgewandelten Verankerungspfeiler 132, welcher kegelstumpfförmige Verankerungsbunde 134 aufweist. Ein solcher Verankerungspfeiler ist besonders gut in derjenigen Richtung belastbar, in welcher die Kräfte auf die breite Stirnfläche der Verankerungsbunde 134 ausgerichtet werden. Je nachdem, ob beim jeweils betrachteten Verankerungspfeiler oder Verankerungspfeilerabschnitt (es versteht sich, daß man einen Verankerungspfeiler auch nur teilweise mit kegelförmigen Verankerungsbunden besetzen kann, im übrigen mit zylindrischen Verankerungsbunden, oder daß man auf einem einzigen Verankerungspfeiler auch unterschiedlich ausgerichtete kegelförmige Verankerungsbunde vorsehen kann) die breiten Seiten der Verankerungsbunde nach oben oder unten weisen, kann der Verankerungspfeiler 132 im Prothesenteil in besonderer Weise für die Aufnahme von Druckbelastungen (breite Basisfläche weist nach oben) oder für die Aufnahme von Zugbelastungen (breite Basisfläche weist nach unten) geeignet sein.

Figure 12 zeigt einen Ausschnitt aus einem Verankerungspfeiler 136 der aus zwei ineinandergeschachtelten Sätzen zylindrischer Pfeilerabschnitte 138, 140 besteht, deren Achsen unter Abstand parallel verlaufen. Man erhält so sichelförmige Schultern 142, welche die Funktion der kreisringförmigen Stirnflächen der oben beschriebenen Formen von Verankerungsbunden übernehmen. Der Verankerungspfeiler 136 ist ein einstückiges Guß- oder Schmiedeteil.

Bei einem weiteren Verankerungspfeiler 144 nach Figur 13 sind auf einen glatten Kern Verankerungskugeln 146 fest aufgebracht, z.B. angegossen, die vom Knochenmaterial umwachsen werden können.

Figur 14 zeigt einen z.B. aus Titan geschmiedeten Verankerungspfeiler 150 mit parallel unter transversalem Abstand verlaufenden Pfeilerbaschnitten 152 und 154, welche durch transversale Pfeilerabschnitte 156 verbunden sind.

Der in Figur 15 gezeigte Verankerungspfeiler 158 ist ein rinnenförmiges Blechbiegteil mit einer Bodenwand 160 und Seitenwänden 162. In diese Wände sind in transversaler Richtung verlaufende Sicken 164 eingeprägt, welche sowohl mit ihrer Außenseite als auch mit ihrer Innenfläche Haltemöglichkeiten für das Knochenmaterial bereitstellen.

Auch der Verankerungspfeiler 160 nach Figur 16 ist ein rinnenförmiges Blechbiegeteil, welches nun aber halbkreisförmigen Querschnitt aufweist und in welches Sicken 168 eingedrückt sind, deren Breite etwa dem Sickenabstand entspricht. Auch hier bieten Außenfläche und Innenfläche Schultern, gegen welche das nachwachsende Knochenmaterial anliegen kann.

Den oben beschriebenen verschiedenen Ausführungsformen von Verankerungspfeilern ist gemeinsam, daß ihre Achse bzw. ihre Haupterstreckungsrichtung im Prothesenteil längs einer der Hauptspannungsrichtungen im Knochen verläuft und daß sie eine große Anzahl von radialen Flächen vorgeben, welche eine axiale Relativbewegung zwischen dem Verankerungspfeiler und dem an es herangewachsenen, regenerierten Knochenmaterial verhindern und jeweils einen kleinen Teil der von der Prothese auf das Knochenende zu übertragenden Kräfte einerseits schonend anderserseits zur natürlichen laufenden Regenerierung anregend auf das benachbarte Spongiosavolumen übertragen.

Die Erfindung wurde obenstehend unter Bezugnahme auf solche Prothesenteile beschrieben, welche als Teil einer Hüftgelenksprothese am oberen Ende des Oberschenkelknochens befestigt werden. Es versteht sich, daß die Erfindung gleichermaßen für andere Prothesenteile verwendbar ist, die an anderen Knochen befestigt werden. es sind dies insbesondere Prothesenteile zur Bildung anderer künstlicher Gelenke, z.B. Ellbogengelenk, Kniegelenk, Fingergelenke usw.

## Ansprüche

1. Orthopädisches Prothesenteil mit einem Gelenkelement (26, 28) zur Zusammenarbeit mit einem komplementären Gelenkelement eines anderen Prothesenteiles und mit einem das Gelenkelement tragenden Verankerungsteil, welches fest mit einem Abschnitt eines Skelettknochens verbindbar ist und eine Tragwand (20) aufweist, auf deren einer Seite das Gelenkelement (26) angeordnet ist, von der anderen Seite der Tragwand im Abstand voneirander mehrere einsele, gekrümmte, stabförmige Verankerungspfeiler (30) ausgenen dadurch gekennzeichnet, daß ; daß die Verankerungspfeiler (30) jeweils eine Vielzahl in axialer Richtung aufeinander folgender Verankerungsbunde (32) tragen ; und daß die Verankerungsbunde (30) so angeordnet und gekrümmt sind, daß die sich im wesentlichen längs hauptkraftübertragungswegen des Spongiosamateriales im betrachteten Skelettknochenabschnitt (10) erstrecken.

2. Prothesenteil nach Anspruch 1, dadurch gekennzeichnet, daß die axiale Abmessung der Verankerungsbunde (32) klein gegenüber dem Abstand aufeinanderfolgender Verankerungsbunde (32) auf einem der Verankerungspfeiler (30) ist.

3. Prothesenteil nach Anspruch 1 oder 2, dadurch gekennzeichnet, daß der Rand der Verankerungsbunde (32) abgerundet ist.

4. Prothesenteil nach einem der Ansprüche 1 bis 3, dadurch gekennzeichnet, daß die Gesamtzahl der Verankerungspfeiler (30), deren Länge, Querschnittsform und Querschnittsabmessung, die Anzahl der jeweils auf den Verankerungspfeilern (30) angeordneten Verankerungsbunde (32), deren Form und Abmessungen so gewählt sind, daß die lokalen Belastungen in der an das Prothesenteil herangewachsenen Spongiosa bei kurzfristigen Arbeitsbelastungen etwa 120% bis etwa 140% der Druckfließgrenze der Spongiosa erreichen, während die lokalen Belastungen der Spongiosa bei Langzeitbelastung bei etwa 60% bis etwa 80% der Druckfließgrenze bleiben.

5. Prothesenteil nach Anspruch 4, dadurch gekennzeichnet, daß die Gesamtoberfläche der Verankerungspfeiler (30) etwa 130% bis etwa 270% der Wandfläche des Hohlraumes (14) beträgt, der zur Implantierung des Prothesenteiles zunächst ausgeräumt werden muß.

6. Prothesenteil nach einem der Ansprüche 1 bis 5, dadurch gekennzeichnet, daß die Verankerungsbunde (32) unmittelbar benachbarter Verankerungspfeiler (30) gegeneinander versetzt sind.

7. Prothesenteil nach einem der Ansprüche 1 bis 6, dadurch gekennzeichnet, daß Zwischenräume, die zwischen benachbarten Verankerungspfeilern (30) verbleiben, im wesentlichen gleich große Querschnittsfläche haben.

8. Prothesenteil nach einem der Ansprüche 1 bis 5, dadurch gekennzeichnet, daß die Tragwand (20) mindestens ein außenliegendes Positionierelement (94) für ein externes auf Zug belastbares Halteelement (42) aufweist.

9. Prothesenteil nach einem der Ansprüche 1 bis 8, dadurch gekennzeichnet, daß die Tragwand (20) so geformt ist, daß sie einen die Kompakta des betrachteten Skelettknochenabschnittes (10) bündig verschließenden Deckel darstellt.

10. Prothesenteil nach einem der Ansprüche 1 bis 9, dadurch gekennzeichnet, daß die Endabschnitte der Verankerungspfeiler (30) freitragend sind.

11. Prothesenteil nach einem der Ansprüche 1-9, dadurch gekennzeichnet, daß Endabschnitte einiger der Verankerungspfeilern (30) verbunden sind.

12. Prothesenteil nach Anspruch 11, dadurch gekennzeichnet, daß unter den Verankerungspfeilern solche (102-108) sind, welche einen sich verjüngenden Käfig bildend von der Tragwand (20) weglaufen und an ihren Enden durch ein olivenförmiges Endstück (110) mit im wesentlichen glatter Außenfläche verbunden sind.

13. Prothesenteil nach Anspruch 12, dadurch gekennzeichnet, daß das Endstück (110) eine axiale Durchgangsbohrung (111) aufweist.

14. Prothesenteil nach einem der Ansprüche 1-13, dadurch gekennzeichnet, daß die Tragwand (20) primäre lange Verankerungspfeiler (102-108) mit großem Querschnitt und kurze Verankerungspfeiler (124) mit kleinem Querschnitt trägt, wobei die Verankerungspfeiler (102-108) mit großem Querschnitt unter großem Abstand voneinander und die Verankerungspfeiler (124) mit kleinem Querschnitt unter kleinem Abstand voneinander angeordnet sind.

15. Prothesenteil nach Anspruch 14, dadurch gekennzeichnet, daß die auf der Tragwand (20) angeordneten primären Verankerungspfeiler (102-108) mit großem Querschnitt und/oder diese primären Verankerungspfeiler (102-108) verbindende, in Umfangsrichtung verlaufende, große Querschnitte aufweisende Verankerungspfeiler (114-120) ihrerseits kurze Verankerungspfeiler (124) mit kleinem Querschnitt tragen, die sich jeweils vom großen Querschnitt aufweisenden Verankerungspfeiler im wesentlichen in Richtung von der Tragwand (20) weg erstrecken.

16. Prothesenteil nach Anspruch 14 oder 15, dadurch gekennzeichnet, daß die kurzen, kleine Querschnitte aufweisenden Verankerungspfeiler (124) einen durch die große Querschnitte aufweisenden Verankerungspfeiler (102-108, 114-120) in seiner Außenkontur vorgegebenen Käfig im wesentlichen gleichförmig belegen.

17. Prothesenteil nach einem der Ansprüche 14-16, dadurch gekennzeichnet, daß die große Durchmesser aufweisenden Verankerungspfeiler (102-108, 114-120) etwa folgende Abmessungen haben : Kerndurchmesser etwa 3,0-4,5 mm, Durchmesser der Verankerungsbunde (122) etwa 0,8-1,5 mm größer als der Kerndurchmesser, axiale Erstreckung der Verankerungsbunde (122) etwa 0,6-1,2 mm, Abstand der Verankerungsbunde (122) voneinander etwa 3-6 mm ; und daß die kleinen Verankerungspfeiler (124) etwa folgende Abmessungen aufweisen : Kerndurchmesser etwa 1,5-3 mm, Durchmesser der Verankerungsbunde etwa 0,6-1,2 mm größer als Kerndurchmesser, axiale Abmessung der Verankerungsbunde etwa 0,5-1 mm, Abstand der Verankerungsbunde etwa 2-4 mm.

18. Prothesenteil nach einem der Ansprüche 1-17, dadurch gekennzeichnet, daß unter den Verankerungspfeilern solche (132) sind, die trapezförmige axiale Querschnitte aufweisende, Kegelförmige Verankerungsbunde (134) haben, wobei einige der kegelförmigen Verankerungsbunde (134) mit ihrer großen Basisfläche und andere der Verankerungsbunde (134) mit ihrer kleinen Stirnfläche zur Tragwand (20) weisen.

19. Verfahren zum Herstellen eines Prothesenteiles nach einem der Ansprüche 1 bis 18, dadurch gekennzeichnet, daß von dem Prothesenteil ein maßgenaues Modell hergestellt wird, und unter Verwendung dieses Modelles eine Negativ-Gießform hergestellt wird, in diese Gießform eine flüssige Prothesenlegierung wie das Biomaterial CoCrMo gegossen wird, und der gegossene Prothesenrohling ausgeformt, gegebenenfalls entgratet, gereinigt sowie gegebenenfalls oberflächenbehandelt wird.

20. Verfahren nach Anspruch 19, dadurch gekennzeichnet, daß das Prothesenmodell aus einem Material hergestellt wird, welches bei einer Temperatur schmilzt oder thermisch zersetzbar ist, durch welche die Negativ-Gießform nicht beeinträchtigt wird.

21. Verfahren nach Anspruch 19 oder 20, dadurch gekennzeichnet, daß das Prothesenmodell in ein maßgenaues Modell des betrachteten Skelettknochenabschnittes eingepaßt wird, wobei das Knochenmodell unter Verwendung von Röntgenaufnahmen unter Berücksichtigung des auszuräumenden Spongiosavolumens hergestellt wird.

22. Verfahren nach Anspruch 21, dadurch gekennzeichnet, daß zur Herstellung des Knochenmodelles zumindest zwei in unterschiedlichen, vorzugsweise zueinander senkrechten Richtungen aufgenommene Röntgenaufnahmen verwendet werden.

23. Verfahren nach Anspruch 22, dadurch gekennzeichnet, daß die Randkonturen des Bildes des Skelettknochens auf den Röntgenaufnahmen und die Randkonturen des auszuräumenden Spongiosavolumens sowie die Randkonturen zu entfernender Teile der Kompakta digitalisiert und in einen Rechner eingegeben werden, welcher unter Verwendung dieser digitalen Daten eine numerisch gesteuerte Werkzeugmaschine ansteuert, um das Knochenmodell aus einem Rohling herauszuarbeiten.

24. Verfahren nach Anspruch 23, dadurch gekennzeichnet, daß dem Rechner ein Massenspeicher zugeordnet ist, bei welchem eine Vielzahl digitalisierter dreidimensionaler Bilder unterschiedlicher Typen für den betrachteten Skelettknochen abgelegt ist, und daß der Rechner diese abgespeicherten Knochenformtypen mit den eingegebenen Knochenrandkonturen vergleicht, unter Durchführen von linearen Ähnlichkeitstransformationen den am besten mit den digitalisierten Randkonturen zusammenpassenden Knochenformtyp auswählt und primär unter Verwendung der digitalisierten Randkonturen und bezüglich der hieraus nicht ableitbaren geometrischen Einzelheiten bezugnehmend auf den ausgesuchten Knochenformtyp die Herstellung des Knochenmodelles steuert.

25. Verfahren nach Anspruch 23 oder 24, dadurch gekennzeichnet, daß der Rechner die Herstellung eines Modelles für die Tragwand des Prothesenteiles derart steuert, daß das Tragwandmodell in seinen Randabschnitten bündig in das Knochenmodell übergeht.

26. Verfahren nach Anspruch 25, dadurch gekennzeichnet, daß der Rechner unter Berücksichtigung der im Massenspeicher für den betrachteten Knochenformtyp mit abgelegten Hauptkraftübertragungswege und unter Berücksichtigung des auszuräumenden Spongiosavolumens und des Querschnittes der Verankerungspfeiler sowie des gewünschten Füllungsgrades des ausgeräumten Spongiosavolumens mit Verankerungspfeilern einen Pfeilerbelegungsplan für das Spongiosavolumen erstellt und graphisch ausgibt.

27. Verfahren nach Anspruch 26, dadurch gekennzeichnet, daß unter Steuerung des Rechners an den Ansatzstellen der Pfeilermodelle am Tragwandmodell geneigte Aufnahmebohrungen für die Pfeilermodelle erzeugt werden.

## Claims

1. Orthopaedic prosthetic device with a joint member (26, 28) for co-operation with a complementary joint memher of another prosthetic device and with an anchoring part supporting the joint member, which anchoring part can be securely connected to a section of a bone of the skeleton and comprises a supporting wall (20), on one side of which the joint member (26) is located, characterised in that starting from the other side of the supporting wall and spaced apart from each other are several individual, curved, rod-like anchoring posts (30) ; that the anchoring posts (30) each support a plurality of anchoring collars (32) following each other in the axial direction ; and that the anchoring posts (30) are arranged and curved so that they extend substantially along main force-transmission paths of the spongiosa material in the skeleton bone section (10) in question.

2. Prosthetic device according to Claim 1, characterised in that the axial dimension of the anchoring collars (32) is small in comparison with the spacing of successive anchoring collars (32) on one of the anchoring posts (30).

3. Prosthetic device according to Claim 1 or 2, characterised in that the edge of the anchoring collars (32) is rounded.

4. Prosthetic device according to one of Claims 1 to 3, characterised in that the total number of anchoring posts (30), their length, cross-sectional shape and cross-sectional dimensions, the number of anchoring collars (32) respectively located on the anchoring posts (30), their shape and dimensions are chosen so that in the case of working loads of short duration, the local loads in the spongiosa which has grown onto the prosthetic device reach approximately 120% to approximately 140% of the compression flow limit of the spongiosa, whereas in the case of long-term loads, the local loads on the spongiosa remain at approximately 60% to approximately 80% of the compression flow limit.

5. Prosthetic device according to Claim 4, characterised in that the total surface of the anchoring posts (30) amounts to approximately 130% to approximately 270% of the wall surface of the cavity (14), which must first of all be cleaned out for the implantation of the prosthetic device.

6. Prosthetic device according to one of Claims 1 to 5, characterised in that the anchoring collars (32) of directly adjacent anchoring posts (30) are staggered with respect to each other.

7. Prosthetic device according to one of Claims 1 to 6, characterised in that gaps, which remain between adjacent anchoring posts (30), have a cross-sectional area of substantially the same size.

8. Prosthetic device according to one of Claims 1 to 5, characterised in that the supporting wall (20) comprises at least one external positioning member (94) for an external retaining member (42), which can be subject to tension.

9. Prosthetic device according to one of Claims 1 to 8, characterised in that the supporting wall (20) is shaped so that it represents a cover closing off in a flush manner the compact bone of the skeleton bone section (10) in question.

10. Prosthetic device according to one of Claims 1 to 9, characterised in that the end sections of the anchoring posts (30) are self-supporting.

11. Prosthetic device according to one of Claims 1 to 9, characterised in that end sections of some of the anchoring posts (30) are connected.

12. Prosthetic device according to Claim 11, characterised in that included amongst the anchoring posts are those (102-108), which extend away from the supporting wall (20) with the formation of a tapering cage and at their ends are connected by an olive-shaped end piece (110) with a substantially smooth outer surface.

13. Prosthetic device according to Claim 12, characterised in that the end piece (110) comprises an axial throughhole (111).

14. Prosthetic device according to one of Claims 1 to 13, characterised in that the supporting wall (20) supports primary, long anchoring posts (102-108) of large cross-section and short anchoring posts (124) of small cross-section, the anchoring posts (102-108) of large cross section being arranged at a great distance apart and the anchoring posts (124) of small cross-section being arranged at a small distance apart.

15. Prosthetic device according to Claim 14, characterised in that the primary anchoring posts (102-108) of large cross-section located on the supporting wall (20) and/or anchoring posts (114-120) of large cross-section, extending in the peripheral direction and connecting these primary anchoring posts (102-108) in turn support short anchoring posts (124) of small cross-section, which each extend away from the anchoring post of large cross-section substantially in the direction of the supporting wall (20).

16. Prosthetic device according to Claim 14 or 15, characterised in that the short anchoring posts (124) having a small cross-section occupy substantially uniformly a cage defined in its outer contour by the anchoring posts (102-108, 114-120) of large cross-section.

17. Prosthetic device according to one of Claims 14 to 16, characterised in that the anchoring posts (102108, 114-120) having a large diameter have approximately the following dimensions : core diameter approximately 3.0-4.5 mm, diameter of the anchoring collars (122) approximately 0.8-1.5 mm larger than the core diameter, axial extent of the anchoring collars (122) approximately 0.61.2 mm, spacing of the anchoring collars (122) from each other approximately 3-6 mm ; and that the small anchoring posts (124) have approximately the following dimensions : core diameter approximately 1.5-3 mm, diameter of the anchoring collars approximately 0.6-1.2 mm larger than the core diameter, axial dimension of the anchoring collars approximately 0.5-1 mm, spacing of the anchoring collars approximately 2-4 mm.

18. Prosthetic device according to one of Claims 1 to 17, characterised in that included amongst the anchoring posts are those (132), which have conical anchoring collars (134) having trapezoidal, axial cross-sections, some of the conical anchoring collars (134) pointing towards the supporting wall (20) with their large base surface and others of the anchoring collars (134) pointing towards the supporting wall (20) with their small end face.

19. Method for the manufacture of a prosthetic part according to one of Claims 1 to 18, characterised in that a model which is true to size is made of the prosthetic device and using this model a negative mould is produced, a liquid prosthetic alloy such as the biomaterial CoCrMo is poured into this mould and the cast prosthetic blank is removed from the mould, burrs are possibly removed therefrom, it is cleaned and if necessary surface-treated.

20. Method according to Claim 19, characterised in that the prosthetic model is made from a material which melts or may be decomposed thermally at a temperature at which the negative mould is not damaged.

21. Method according to Claim 19 or 20, characterised in that the prosthetic model is fitted in a true-to-size model of the skeleton bone section in question, the bone model being produced using X-ray photographs, taking into consideration the volume of spongiosa to be cleaned out.

22. Method according to Claim 21, characterised in that for producing the bone model, at least two X-ray photographs taken in different directions, preferably at right angles to each other, are used.

23. Method according to Claim 22, characterised in that the marginal contours of the image of the skeleton bone on the X-ray photographs and the marginal contours of the volume of spongiosa to be cleaned out as well as the marginal contours of the parts of the compact bone to be removed are converted into digital form and fed into a computer, which with the use of these digital data controls a numerically controlled machine tool, in order to rough-machine the bone model from a blank.

24. Method according to Claim 23, characterised in that associated with the computer is a dimension memory, in which a plurality of digitalized, three-dimensional images of different types is stored for the skeleton bone in question and that the computer compares these memorised bone shape types with the marginal contours of the bone fed in, whilst carrying out linear similarity transformations, selects the bone shape type, which is the best match with the digitalized marginal contours and primarily, using the digitalized marginal contours and with regard to the geometric details which cannot be derived herefrom, referring to the selected bone shape type, controls the production of the bone model.

25. Method according to Claim 23 or 24, characterised in that the computer controls the production of a model for the supporting wall of the prosthetic device so that in its marginal sections, the supporting wall model passes flush into the bone model.

26. Method according to Claim 25, characterised in that taking into consideration the main force-transmission paths memorised in the dimension memory for the bone shape type in question and taking into consideration the volume of spongiosa to be cleaned out and of the cross-section of the anchoring posts as well as of the desired degree of filling of the volume of spongiosa removed with anchoring posts, the computer produces a post occupancy plan for the spongiosa volume and displays it graphically.

27. Method according to Claim 26, characterised in that by controlling the computer, inclined receiving bores for the post models are produced at the attachment points of the post models to the supporting wall model.

**Revendications**

1. Dispositif prothétique orthopédique comportant un élément d'articulation (26, 28) prévu pour coopérer avec un élément d'articulation complémentaire d'une autre partie de prothèse et une pièce d'ancrage portant l'élément d'articulation (26, 28), laquelle peut être reliée de manière fixe à une partie d'un os du squelette et présente une paroi d'appui (20) d'un côté de laquelle se trouve disposé l'élément d'articulation (26), caractérisé en ce que de l'autre côté de la paroi d'appui (20) se projettent plusieurs piliers d'ancrage séparés (30) en forme de tige courbée, agencés de manière écartée les uns des autres ; en ce que les piliers d'ancrage (30) comportent chacun une pluralité de segments d'ancrage (32) se suivant dans le sens axial ; et en ce que les piliers d'ancrage (30) sont disposés et courbés de telle sorte qu'ils s'étendent substantiellement le long de voies principales de transmission de forces du tissu spongieux au sein de la partie concernée de l'os (10) du squelette.

2. Dispositif suivant la revendication 1, caractérisé en ce que la dimension axiale des segments d'ancrage (32) est petite par rapport à la distance entre les segments d'ancrage (32) successifs d'un des piliers d'ancrage (30),

3. Dispositif suivant la revendication 1 ou 2, caractérisé en ce que le bord des segments d'ancrage (32) est arrondi.

4. Dispositif suivant une des revendications 1 à 3, caractérisé en ce que le nombre total de piliers d'ancrage (30), leur longueur, leur forme transversale et leur diamètre, ainsi que le nombre de segments d'ancrage (32) de chacun des piliers d'ancrage (30), leur forme et leurs dimensions sont définis de telle sorte que les charges locales au sein du tissu spongieux entourant les piliers d'ancrage (30) atteignent environ 120% à environ 140% de la limite d'élasticité par compression du tissu spongieux en présence de charges de travail de courte durée, tandis que lors d'une charge de travail de longue durée, les charges locales au sein du tissu spongieux demeurent entre environ 60% à environ 80% de la limite d'élasticité par compression.

5. Dispositif suivant la revendication 4, caractérisé en ce que la surface totale des piliers d'ancrage (30) représente environ 130% à environ 270% de la surface de la paroi de la cavité (14), laqualle doit d'abord être vidée afin d'y implanter le dispositif prothétique.

6. Dispositif suivant une des revendications 1 à 5, caractérisé en ce que les segments d'ancrage (32) de piliers d'ancrage (30) directement voisins sont décalés les uns par rapport aux autres.

7. Dispositif suivant une des revendications 1 à 6, caractérisé en ce que les espaces subsistant entre des piliers d'ancrage (30) voisins occupent des surfaces transversales substantiellement égales.

8. Dispositif suivant une des revendications 1 à 5, caractérisé en ce que la paroi d'appui (20) présente un élément de positionnement extérieur (94) au moins destiné à un élément de maintien externe (42) soumis à une contrainte de traction.

9. Dispositif suivant une des revendications 1 à 8, caractérisé en ce que la paroi d'appui (20) est conçue de telle sorte qu'elle forme un couvercle obturant exactement le tissu compact de la partie concernée de l'os (10) du squelette.

10. Dispositif suivant une des revendications 1 à 9, caractérisé en ce que les extrémités des piliers d'ancrage (30) sont libres.

11. Dispositif suivant une des revendications 1 à 9, caractérisé en ce que les extrémités de quelques uns des piliers d'ancrage (30) sont reliées.

12. Dispositif suivant la revendication 11, caractérisé en ce que parmi les piliers d'ancrage certains (102 - 108) s'éloignent de la paroi d'appui (20) en formant une cage se rétrécissant et sont reliés à leurs extrémités au moyen d'un embout (110) en forme d'olive présentant une surface extérieure substantiellement lisse.

13. Dispositif suivant la revendication 12, caractérisé en ce que l'embout (110) présente un orifice de passage axial (111).

14. Dispositif suivant une des revendications 1 à 13, caractérisé en ce que la paroi d'appui (20) porte des piliers d'ancrage primaires longs (102 - 108) de forte section et des piliers d'ancrage courts (124) de faible section, les piliers d'ancrage (102 - 108) de forte section étant disposés à une distance importante les uns des autres et les piliers d'ancrage (124) de faible section étant disposés à une faible distance les uns des autres.

15. Dispositif suivant la revendication 14, caractérisé en ce que les piliers d'ancrage primaires (102 - 108) de forte section disposés sur la paroi d'appui (20) et/ou les piliers d'ancrage (114 - 120) de forte section prévus dans le sens périphérique et reliant lesdits piliers primaires (102 - 108) portant de leur côté des piliers d'ancrage courts (124) de faible section, lesquels s'étendent chaque fois depuis les piliers d'ancrage de forte section substantiellement an direction de la paroi d'appui(20).

16. Dispositif suivant la revendication 14 ou 15, caractérisé en ce que les piliers d'ancrage courts (124) de faible section garnissent de manière substantiellement homogène un panier, dont le contour extérieur est défini par les piliers d'ancrage (102108, 114 - 120) de forte section.

17. Dispositif suivant une des revendications 14 à 16, caractérisé en ce que les piliers d'ancrage (102 - 108, 114 - 120) de forte section possèdent à peu près las dimensions suivantes : diamètre de l'âme environ 3,0 à 4,5 mm, diamètre des segments d'ancrage (122) environ 0,8 à 1,5 mm de plus que le diamètre de l'âme, dimension axiale des segments d'ancrage (122) environ 0,6 à 1,2 mm, écartement des segments d'ancrage (122) environ 3 à 6 mm ; et en ce que les piliers d'ancrage (124) de faible section possèdent à peu près les dimensions suivantes : diamètre de l'âme environ 1,5 à 3,0 mm, diamètre des segments d'ancrage environ 0,6 à 1,2 mm de puis que le diamètre de l'âme, dimension axiale des segments d'ancrage environ 0,5 à 1 mm, écartement des segments d'ancrage environ 2 à 4 mm.

18. Dispositif suivent une des revendications 1 à 17, caractérisé en ce que parmi les piliers d'ancrage certains (132) possèdent des segments d'ancrage coniques (134) présentant une section axiale trapézoïdale, quelques uns des segments d'ancrage coniques (134) étant disposés avec leur grande base orientée vers la paroi d'appui (20) et quelques autres des segments d'ancrage coniques (134) étant disposés avec leur petite base orientée vers le paroi d'appui (20).

19. Procédé de fabrication d'un dispositif prothétique suivant une des revendications 1 à 18, caractérisé en ce qu'un modèle aux dimensions exactes du dispositif prothétique est fabriqué et un moule négatif réalisé en se servant dudit modèle, et en ce qu'un alliage liquide pour prothèses, telle que la biomatière CoCrMo, est coulé dans ledit moule et l'ébauche de prothèse est démoulée, le cas échéant ébarbée, nettoyée, ainsi que traitée en surface le cas échéant.

20. Procédé suivant la revendication 19, caractérisé en ce que le modèle de la prothèse est fabriqué dans une matière fondant ou se désintégrant thermiquement à une température n'influençant pas le moule négatif.

21. Procédé suivant la revendication 19 ou 20, caractérisé en ce que le modèle de la prothèse est adapté de manière à obtenir un modèle aux dimensions exactes de la partie concernée de l'os du squelette, le modèle de l'os étant réalisé en utilisant des radiographies en tenant compte du volume de tissu spongieux à enlever.

22. Procédé suivant la revendication 21, caractérisé en ce que deux radiographies au moins prises sous des angles différents, de préférence perpendiculaires, sont utilisées pour la fabrication du modèle de l'os.

23. Procédé suivent la revendication 22, caractérisé en ce que le contour de l'os du squelette sur les radiographies et le contour du volume de tissu spongieux à enlever ainsi que le contour de la partie de tissu compact à enlever sont numérisés et introduits dans un ordinateur, lequel exploite ces données numériques pour commander une machine-outil à commande numérique, afin de façonner le modèle de l'os à partir d'une ébauche.

24. Procédé suivant la revendication 23, caractérisé en ce que l'ordinateur est relié à une mémoire centrale, au sein de laquelle se trouve stockée une pluralité d'images tridimensionnelles numérisées de différentes formes types de l'os concerné du squelette et en ce que l'ordinateur compare ces formes types mémorisées avec le contour de l'os introduit, sélectionne la forme type de l'os correspondant le mieux au contour numérisé en procédant à des transformations de similitudes linéaires et commande la fabrication du modèle de l'os en se servant avant tout du contour numérisé de l'os et en se référant à la forme type sélectionnée de l'os, en ce qui concerne les particularités géométriques ne pouvant être déduites du contour numérisé.

25. Procédé suivant la revendication 23 ou 24, caractérisé en ce que l'ordinateur commande la fabrication d'un modèle de la paroi d'appui de la prothèse de telle sorte que le modèle de la paroi d'appui se raccorde exactement au modèle de l'os selon son contour.

26. Procédé suivant la revendication 25, caractérisé e n ce que l'ordinateur établit et édite graphiquement un plan d'implantation des piliers d'ancrage pour le volume de tissu spongieux, en tenant compte des voies principales de transmission de forces stockées dans la mémoire centrale en fonction des formes types de l'os concerné et en tenant compte du volume de tissu spongieux à enlever, de la section des piliers d'ancrage ainsi que du degré de remplissage souhaité en piliers d'ancrage du volume de tissu spongieux enlevé.

27. Procédé suivant la revendication 26, caractérisé en ce que sous la commande de l'ordinateur, des trous de positionnement inclinés destinés aux modèles de piliers d'ancrage sont réalisés dans le modèle de la paroi d'appui, aux endroits correspondant à la base des modèles de piliers.

Fig. 1

Fig.2

EP 0 245 846 B1

Fig. 3

18

Fig. 4

Fig. 5

80

Fig. 6

82    76

88

86

Fig. 7

88

86

90

Fig. 8

Fig. 9

18″

28

22  20

26

130

126

108

128

112

120

122

102

110

124

118

116

114

106

104

111

*Fig. 10*

132  134

*Fig. 11*

Fig. 12

Fig 13

Fig. 14

Fig. 15

Fig. 16